# EUROPEAN PATENT SPECIFICATION

(11) **EP 1 426 051 B1**
(45) Date of publication and mention of the grant of the patent: **16.07.2008**
(21) Application number: 02751784.6
(22) Date of filing: 30.07.2002
(51) Int. Cl.: A61K 31/551, A61K 45/00, A61P 9/10, A61P 25/28

(54) **MEDICINAL COMPOSITION FOR PREVENTION OF OR TREATMENT FOR CEREBROVASCULAR DISORDER**
MEDIZINISCHE ZUSAMMENSETZUNG ZUR PRÄVENTION ODER BEHANDLUNG VON ZEREBROVASKULÄREN STÖRUNGEN
COMPOSITION MEDICINALE POUR LA PREVENTION OU LE TRAITEMENT DES TROUBLES VASCULAIRES CEREBRAUX

(30) Priority: 11.09.2001 JP 2001274846
(43) Date of publication of application: 09.06.2004
(73) Proprietor: Asahi Kasei Pharma Corporation, Tokyo 101-8481 (JP)
(72) Inventor: TOSHIMA, Yoshinori, Tagata-gun, Shizuoka 410-2124 (JP); HITOMI, Asako, Tagata-gun, Shizuoka 419-0114 (JP); SATOH, Shin-ichi, Tagata-gun, Shizuoka 410-2221 (JP); IKEGAKI, Ichiro, Tagata-gun, Shizuoka 419-0113 (JP)
(74) Representative: Forstmeyer, Dietmar
(86) International application number: PCT/JP2002/007712
(87) International publication number: WO 2003/024457

(56) References cited:
- EP-A- 0 845 265
- EP-A- 0 885 888
- EP-B1- 0 187 371
- WO-A1-93/23082
- WO-A2-02/09761
- WO-A2-96/05838
- WO-A2-96/25933
- JP-A- 10 007 590
- JP-A- 2002 226 374
- WATARU MUKAI ET AL.: 'Koreisha ya shinkino teika o gappei shita kyoshinsho no chiryo no kihon' MEDICINA vol. 31, no. 13, 1994, pages 2571 - 2573, XP002958942

## Description

### Technical Field

The present invention relates to a pharmaceutical composition that is effective against prevention or treatment of diseases such as a cerebrovascular disorder.

### Related Art

For prevention or treatment of diseases such as a cerebrovascular disorder, there have been used drugs such as an ameliorant of cerebral circulation, a vasodilator, a cerebral protecting drug, an brain metabolic stimulants, an anticoagulant, an antiplatelet drug, a thrombolytic drug, an amelirant of psychiatric symptom, a antihypertensive drug, a diuretic, an antihyperlipidemic drug, and an immunosuppressant.

On the other hand, it has been already known that a compound represented by the general formula (I): 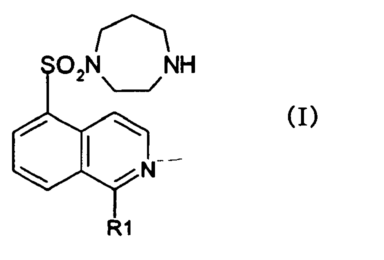 (wherein R1 represents a hydrogen atom) has an inhibitory activity against a kinase such as a Rho kinase, a myosin light chain kinase, or a protein kinase C; shows a vascular smooth muscle relaxation effect, a blood flow increasing effect, an antihypertensive effect, a cerebral protective effect, a cardiac protective effect, or the like; and is an effective substance in a vasodilator agent (particularly as a treating agent for angina), a cerebral protecting agent, a cardiac protecting agent, or the like (for example, JP 61-227581 A, JP 02-256617 A, JP 06-056668 A, JP 07-080854 B, EP 0187371, WO 98/06433, Br. J. Pharmacol., 98, 1091 (1989), J. Pharmacol. Exp. Ther., 259, 738 (1991), Circulation, 96, 4357 (1997), Cardiovasc. Res., 43, 1029 (1999)).

In the aforementioned documents, the single use of the compound represented by the general formula (I) is disclosed. However, there have been no report regarding concomitant use or a plan of concomitant use of the compound with an ameliorant of cerebral circulation, a vasodilator, a cerebral protecting drug, an brain metabolic stimulants, an anticoagulant, an antiplatelet drug, a thrombolytic drug, an amelirant of psychiatric symptom, a antihypertensive drug, a diuretic, an antihyperlipidemic drug, or an immunosuppressant until the pharmaceutical composition of the present invention is disclosed.

### Disclosure of the Invention

An object of the present invention is to provide a pharmaceutical composition that has a marked effect than that obtained when a preventive drug or a treatment drug is administered singly for prevention or treatment of a cerebrovascular disorder.

In view of the above-mentioned circumstances, the inventors of the present invention have found that the compound represented by the general formula (I) exerts particularly significant effect that was not obtained when administering the compound singly for, e.g. , drug effect, safeness, stability, dosage, dosage form, usage, or the like by using the compound in combination with nimodipine or a pharmaceutically acceptable salt thereof. Thus, the inventors have accomplished the present invention based on the knowledge.

That is, the present invention relates to the followings:
(1) A pharmaceutical composition for the prevention or treatment of a cerebrovascular disorder selected from the group consisting of cerebral infarction, cerebral thrombosis, cerebral embolism, cerebral hemorrhage, cerebral vasospasm after subarachnoid hemorrhage, transient ischemic attack, cerebral arteriosclerosis, and cerebral edema, including at least one of components (a) and at least one of components (b) shown in below.
   (a) A compound represented by the following general formula (I) : 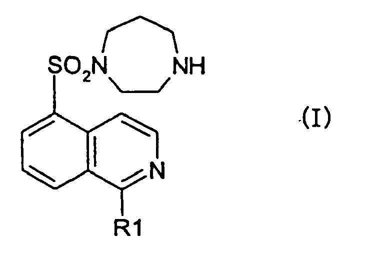 (wherein R1 represents a hydrogen atom) or an acid addition salt or hydrate thereof; and
   (b) nimodipine or a pharmaceutically acceptable salt thereof.
      Note that, in the present invention, the components (a) include, in addition to the compound represented by the general formula (I), hydrates thereof (for example, 1/2 hydrates, 1 hydrates, and 3 hydrates) and acid addition salts thereof.
      Moreover, the drug of components (b), which is used together with the component (a) represented by the general formula (I) according to the present invention, includes pharmaceutically acceptable salts thereof.
(2) The pharmaceutical composition according to item (1), in which the cerebrovascular disorder is cerebral vasospasm developed after subarachnoid hemorrhage.
(3) The pharmaceutical composition according to item (1) or (2), wherein the pharmaceutical composition is a non-mixed combination in form of a kit or a pharmaceutical package.

The compound of the present invention represented by the general formula (I) can be synthesized in accordance with a well known method described in, for example, Chem. Pharam. Bul 1., 40, (3) 770-773 (1992); JP 61-152658 A; or the like. In addition, an acid addition salt thereof is preferably a pharmaceutic ally acceptable nontoxic salt. Examples of the salt include: salts of inorganic acids such as hydrochloric acid, hydrobromic acid, phosphoric acid, and sulfuric acid; and salts of organic acids such as acetic acid, citric acid, tartaric acid, lactic acid, succinic acid, fumaric acid, maleic acid, and methanesulfonic acid.

The pharmaceutical composition of the present invention includes not only a mixture in which at least one of the above-mentioned components (a) and at least one of the above-mentioned components (b) have been mixed previously, but also a nonmixed combination such as a form of a kit or a pharmaceutical package.

On preparing the pharmaceutical composition of the present invention as a formulation having a form suitable for administration, there may be mixed: the compound represented by the general formula (I) (wherein R1 represents a hydrogen atom) provided as an active ingredient or an acid addition salt or hydrate thereof; and (b) nimodipine or a pharmaceutically acceptable salt thereof; and a pharmaceutically acceptable carrier which is known as a supplementary component. Examples of the carrier include: gelatin; saccharides such as lactose and glucose; starches such as wheat, rice, and corn starch; fatty acids such as stearic acid; fatty acid salts such as calcium stearate and magnesium stearate; talc; vegetable oils; alcohols such as stearic alcohol and benzyl alcohol; gum; and polyalkylene glycol.

Moreover, general examples of a liquid carrier include: water; physiological saline; a solution containing dextrose or a similar saccharide; and glycols such as ethylene glycol, propylene glycol, polyethylene glycol, and polypropylene glycol. When the pharmaceutical composition of the present invention is prepared as a capsule, typically, gelatin is preferably used.

The pharmaceutical composition of the present invention, which is composed of: the carrier provided as a supplementary component; the compound represented by the general formula (I) (wherein R1 represents a hydrogen atom) provided as an active ingredient or an acid addition salt or hydrate thereof; and (b) nimodipine or a pharmaceutically acceptable salt thereof, includes an active ingredient in an amount of, typically 0.01 wt% or more and 80 wt% or less, preferably 60 wt% or less, for example.

In addition, a method of treating a cerebrovascular disorder using a certain amount of the compound shown in (a) above and a certain amount of nimodipine (b) or a pharmaceutically acceptable salt thereof. Those may be simultaneously administered as a mixture and may be separately administered successively as a different pharmaceutical form. In the case of the repetitive administration, the time interval between administrations is preferably 48 hours or less. Moreover, the compound shown in (a) and the nimodipine (b) may have different administration routes from each other.

That is, when one has an oral administration, and the other may have a parenteral administration.

Examples of the administration method include oral administration and parenteral administration. Examples of a dosage form suitable for the oral administration include a tablet, a capsule, a powder, a granule, a liquid agent, and an elixir.

Examples of a dosage form suitable for the parenteral administration include a liquid agent.

When the pharmaceutical composition is parenterally administered by intramuscular injection, intravenous injection, or subcutaneous injection, the composition can be administered in the form of a sterile solution that is added with another solute such as sodium chloride or glucose. When the pharmaceutical composition is administered by injection, the composition is preferably dissolved in sterilized water, a lidocaine hydrochloride solution (for intramuscular injection), physiological saline, glucose, a solution for intravenous injection, or an electrolyte solution (for intravenous injection).
When the composition is dissolved in such a manner, the solution may be regulated so as to contain an active ingredient in an amount of, typically 0.01 wt% or more and 20 wt% or less, preferably 0.1 wt% or more and 10 wt% or less. In the case of a liquid agent for oral administration, preferable examples include a suspension or syrup that contains an active ingredient in an amount of 0.01 to 20 wt%. Examples of a carrier to be used in such a case include an aqueous diluents such as perfume, syrup, or pharmaceutical micell.

The dosage of the pharmaceutical composition of the present invention depends on age of a patient to be administered, health condition, body weight, degree of symptom, type of treatment if any simultaneous treatment is performed, frequency of treatment, property of desired effect, administration route, or administration plan. Typically, effective dosage of the compound of the present invention represented by the general formula (I) (wherein R1 represents a hydrogen atom) or an acid addition salt or hydrate thereof is 0.01 to 20 mg/kg·day in the case of parenteral administration, and 0.02 to 40 mg/kg·day in the case of oral administration. Effective dosage of nimodipine (b) or a pharmaceutically acceptable salt thereof is typically 0.0001 to 100 mg/kg·day. In addition, effective dosage of nimodipine is 0.1 to 3 mg/kg·day in the case of parenteral administration, and 0.3 to 30 mg/kg·day in the case of oral administration.

The present invention also provides a kit, package, or document for convenient and effective use of the pharmaceutical composition of the present invention. That is, the present invention relates to a medical kit for prevention or treatment of a cerebrovascular disorder, which is characterized by including a first container containing a certain amount of the compound shown in (a) and a second container containing a certain amount of nimodipine (b) or a pharmaceutically acceptable salt thereof. By using such a kit, the present invention facilitates a use of the pharmaceutical composition of the present invention, and also facilitates accurate administration of a suitable active ingredient to a patient with accurate dosage by a physician.

Moreover, other means that facilitate to understand and use the pharmaceutical composition of the present invention include a briefing document and a package. That is, a document which describes the use of a certain amount of the compound shown in (a) together with a certain amount of nimodipine (b) or a pharmaceutically acceptable salt thereof for prevention or treatment of a cerebrovascular disorder; and a package which includes the document. The document and the package which includes the document facilitate to understand and use the pharmaceutical composition of the present invention, and also facilitates accurate administration of a suitable active ingredient to a patient with accurate dosage by a physician.

### Best Mode for carrying out the Invention

The present invention will next be described more specific ally by way of examples, formulation example, and kit example.

### [Example 1] Effect on dog delayed cerebral vasospasm model

An adult mongrel dog was anesthetized with pentobarbital and 5 ml of autologous blood was injected into cisterna magna (on the first day) . On the third day, 5 ml of autologous blood was injected into cisterna magna under thiamyral anesthesia. Before the injection of autologous blood on the first day and on the seventh day, angiographin was injected into vertebral artery, followed by photographing blood vessel of basilar artery. The blood vessel photograph on the seventh day confirmed occurrence of delayed cerebral vasospasm on the photograph. Then, a hydrochloride of the compound represented by the general formula (I) (wherein R1 represents a hydrogen atom) (0.3 mg/kg) and nimodipine (0.1 mg/kg or 1 mg/kg) were separately administered into vein over 30 minutes continuously (separate administration group). Alternatively, a hydrochloride of the compound represented by the general formula (I) (wherein R1 represents a hydrogen atom) (0.3 mg/kg) and nimodipine (0.1 mg/kg) were concomitantly administered into vein over 30 minutes continuously (concomitant administration group). As a control, physiological saline was administered into vein over 30 minutes continuously. After the administration of each drug, the blood vessel was photographed to measure the diameter of basilar artery. Table 1 shows the percentage (%) of the blood vessel diameter after the administration of each drug based on the blood vessel diameter before the injection of autologous blood (on the first day).

**Table 1**

| Compound | Basilar artery diameter (diameter before blood injection: 100%) |
|---|---|
| Physiological saline | 59.9% |
| Hydrochloride of general formula (I) compound (wherein R1 represents a hydrogen atom) 0.3 mg/kg | 60.5% |
| Nimodipine 0.1 mg/kg | 55.7% |
| Nimodipine 1 mg/kg | 58.0% |
| Hydrochloride of general formula (I) compound (wherein R1 represents a hydrogen atom) 0.3 mg/kg + Nimodipine 0.1 mg/kg | 68.8% |

The diameter of basilar artery measured before the drug administration (on the seventh day) decreased to about 60% compared with that measured before the autologous blood injection (on the first day), and occurrence of delayed cerebral vasospasm was confirmed. When the hydrochloride of the compound represented by the general formula (I) (wherein R1 represents a hydrogen atom) (0.3 mg/kg) and nimodipine (0.1 mg/kg or 1 mg/kg) were separately administered, the vasospasm was not improved. When the hydrochloride of a compound represented by the general formula (I) (wherein R1 represents a hydrogen atom) (0.3 mg/kg) and nimodipine (0.1 mg/kg or 1 mg/kg) were concomitantly administered, an vasospasm improving effect was observed.
Also, an vasospasm improving effect was observed in the case of the concomitant administration of a certain amount of the hydrochloride and nimodipine that had not caused vasospasm improving effect when administering those separately.

Those results confirmed that the concomitant administration of the hydrochloride of the compound represented by the general formula (I) (wherein R1 represents a hydrogen atom) and nimodipine caused a synergistic effect. Also, those results indicated that the concomitant administration of the compound represented by the general formula (I) (wherein R1 represents a hydrogen atom) and nimodipine was effective for improvement and prevention of cerebral vasospasm.

### [Reference Example 2] Inhibitory effect of cerebral infarction in rat cerebral infarction model

A rat brain microthromboembolism model described in Stroke, 31, 2245-2255 (2000) was used as a cerebral infarction model. Physiological saline, a hydrochloride of a compound represented by the general formula (I) (wherein R1 represents a hydrogen atom) (1 mg/kg), or sodium ozagrel provided as an ameliorant of cerebral circulation (10 mg/kg) was separately administered intraperitoneally to a rat provided as a cerebral infarction model (separate administration group) Alternatively, a hydrochloride of a compound represented by the general formula (I) (wherein R1 represents a hydrogen atom) (1 mg/kg) and sodium ozagrel (10 mg/kg) were administered intraperitoneally (concomitant administration group). After the model was prepared, each drug was administered once a day until the fourth day. On the fifth day, the brain was extracted and the size of a cerebral infarction was histopathologically measured. In the case of the separate administration of the hydrochloride of the compound represented by the general formula (I) (wherein R1 represents a hydrogen atom) (1 mg/kg) and sodium ozagrel (10 mg/kg), the cerebral infarction was not inhibited. On the other hand, in the case of the concomitant administration of the hydrochloride of the compound represented by the general formula (I) (wherein R1 represents a hydrogen atom) (1 mg/kg) and sodium ozagrel (10 mg/kg), the cerebral infarction was inhibited. Also, the inhibitory effect of cerebral infarction was confirmed in the case of the concomitant administration of a certain amount of the hydrochloride and sodium ozagrel that had not caused the inhibitory effect of cerebral infarction when administering those separately. It was confirmed that the concomitant administration of the hydrochloride of the compound represented by the general formula (I) (wherein R1 represents a hydrogen atom) and sodium ozagrel caused a synergistic effect. It was indicated that the concomitant administration of the compound represented by the general formula (I) (wherein R1 represents a hydrogen atom) and sodium ozagrel was effective for improvement and prevention of cerebral infarction.

### [Example 3] Effect on neuronal death model by transient occlusion of both common carotid arteries of Mongolian gerbil

Both common carotid arteries of a Mongolian gerbil were occluded for 5 minutes to cause transient brain ischemic condition. Immediately after restarting blood flow, a hydrochloride of the compound represented by the general formula (I) (wherein R1 represents a hydrogen atom) (0.3 mg/kg) or nimodipine (3 mg/kg or 10 mg/kg) was separately administered intraperitoneally (separate administration group).

Alternatively, a hydrochloride of the compound represented by the general formula (I) (wherein R1 represents a hydrogen atom) (0.3 mg/kg) and nimodipine (3 mg/kg) were administered intraperitoneally (concomitant administration group).

On the seventh day, the number of pyramidal cells in a hippocampus CA1 region was counted. The brain ischemia decreased the number of pyramidal cells to about 10%. The separate administration of the hydrochloride of the compound represented by the general formula (I) (wherein R1 represents a hydrogen atom) (0.3 mg/kg) and nimodipine (3 mg/kg) did not inhibit neuronal death. The concomitant administration of the hydrochloride of the compound represented by the general formula (I) (wherein R1 represents a hydrogen atom) (0.3 mg/kg) and nimodipine (3 mg/kg) inhibited neuronal death. Also, the brain inhibitory effect of neuronal death was confirmed in the case of the concomitant administration of a certain amount of the hydrochloride and nimodipine that had not caused the brain inhibitory effect of neuronal death when administering those separately.

Those results confirmed that the concomitant administration of the hydrochloride of the compound represented by the general formula (I) (wherein R1 represents a hydrogen atom) and nimodipine caused a synergistic effect. In addition, those results indicated that the concomitant administration of the compound represented by the general formula (I) (wherein R1 represents a hydrogen atom) and nimodipine was effective for improvement and prevention of cerebral infarction.

### [Reference Example 4] Effect on vasopressin induced rat angina pectoris model

A rat was orally administered with physiological saline, a hydrochloride of the compound represented by the general formula (I) (wherein, R1 is a hydrogen atom) (3 mg/kg), nifedipine provided as a calcium channel blocking drug (3 mg/kg), propranolol provided as a β-adrenaline receptor blocking drug (100 mg/kg), or isosorbide nitrate provided as a nitrate drug (30 mg/kg) separately (separate administration group) . Alternatively, a rat was orally administered with one compound of a hydrochloride of the compound represented by the general formula (I) (wherein, R1 is a hydrogen atom) (3 mg/kg), nifedipine (3 mg/kg), propranolol (100 mg/kg), and isosorbide nitrate (30 mg/kg) (concomitant administration group) . Half an hour later, vasopressin (0.5 U/kg) was intravenously administered. The ST segment depression was used as an index showing the degree of a myocardial ischemia. On the seventh day, the ST segment in an electrocardiogram was measured for comparison with a ST segment before the vasopressin administration. It was found that the vasopressin administration caused the ST segment depression. In addition, occurrence of a myocardial ischemia was observed. The ST segment depression was not improved by the separate administration of the hydrochloride of the compound represented by the general formula (I) (wherein, R1 is ahydrogenatom), nifedipine, propranolol, and isosorbide nitrate. The ST segment depression was improved by the concomitant administration of: the hydrochloride of the compound represented by the general formula (I) (wherein, R1 is a hydrogen atom) and nifedipine; the hydrochloride of the compound represented by the general formula (I) (wherein, R1 is a hydrogen atom) and propranolol; or the hydrochloride of the compound represented by the general formula (I) (wherein, R1 is a hydrogen atom) and isosorbide nitrate. The improving effect of myocardial ischemia was-confirmed in the case of the concomitant administration of a certain amount of the hydrochloride, nifedipine, propranolol, and isosorbide nitrate that had not caused the improving effect of myocardial ischemia when administering those separately. Those results confirmed that the concomitant administration of any of the hydrochloride of the compound represented by the general formula (I) (wherein R1 represents a hydrogen atom), nifedipine, propranolol, and isosorbide nitrate caused a synergistic effect. In addition, those results indicated that the concomitant administration of any of the hydrochloride of the compound represented by the general formula (I) (wherein R1 represents a hydrogen atom), nifedipine, propranolol, and isosorbide nitrate was effective for improvement and prevention of angina pectoris.

### Formulation Example

A pharmaceutical composition, which includes (a), a compound represented by the general formula (I): 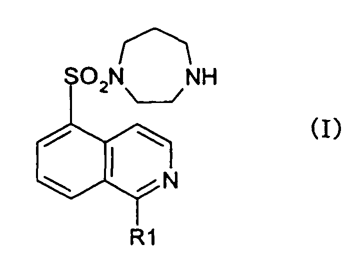 (wherein R1 represents a hydrogen atom) or an acid addition salt or hydrate thereof and which includes (b) can be produced in accordance with, for example, the formulation shown below.

### 1. Sterile injectable

A preferable amount of (b) was added to the components shown in Table 2 below and those compounds were dissolved in distilled water for injection. Subsequently, the solution was regulated to have a required final weight by addition of distilled water for injection and an ampule containing 2 ml of the solution was sealed, followed by heat sterilization.

**[Table 2]**

| - | Component | Active ingredient content |
|---|---|---|
| 10 mg formulation | Hydrochloride of general formula (I) compound (wherein R1 represents a hydrogen atom) | 10 mg |
| | Sodium chloride | 16 mg |
| | Distilled water | Proper amount |
| | | Total amount was regulated to 2 ml |
| 30 mg formulation | Hydrochloride of general formula (I) compound (wherein R1 represents a hydrogen atom) | 30 mg |
| | Sodium chloride | 16 mg |
| | Distilled water | Proper amount |
| | | Total amount was regulated to 2 ml |
| 60 mg formulation | Hydrochloride of general formula (I) compound (wherein R1 represents a hydrogen atom) | 60 mg |
| | Sodium chloride | 16 mg |
| | Distilled water | Proper amount |
| | | Total amount was regulated to 2 ml |
| 10 mg formulation | Hydrochloride of general formula (I) compound (wherein R1 represents a hydroxyl group) | 10 mg |
| | Sodium chloride | 16 mg |
| | Distilled water | Proper amount |
| | | Total amount was regulated to 2 ml |
| 30 mg formulation | Hydrochloride of general formula (I) compound (wherein R1 represents a hydroxyl group) | 30 mg |
| | Sodium chloride | 16 mg |
| | Distilled water | Proper amount |
| | | Total amount was regulated to 2 ml |
| 60 mg formulation | Hydrochloride of general formula (I) compound (wherein, R1 represents a hydroxyl group) | 60 mg |
| | Sodium chloride | 16 mg |
| | Distilled water | Proper amount |
| | | Total amount was regulated to 2 ml |

### 2. Tablet

A preferable amount of (b) was added to the components shown in Table 3 below to prepare a tablet in accordance with a general method.

**[Table 3]**

| | Component | Amount |
|---|---|---|
| 10 mg formulation | Hydrochloride of general formula (I) compound (wherein R1 represents a hydrogen atom) | 10.0 mg |
| | Crystalline cellulose | 25.0 mg |
| | Lactose | 108.5 mg |
| | Magnesium stearate | 1.5 mg |
| | Carboxymethylcellulose calcium | 5.0 mg |
| | Total | 150.0 mg |
| 20 mg formulation | Hydrochloride of general formula (I) compound (wherein R1 represents a hydrogen atom) | 20.0 mg |
| | Crystalline cellulose | 25.0 mg |
| | Lactose | 98.5 mg |
| | Magnesium stearate | 1.5 mg |
| | Carboxymethylcellulose calcium | 5.0 mg |
| | Total | 150.0 mg |
| 10 mg formulation | Hydrochloride of general formula (I) compound (wherein R1 represents a hydroxyl group) | 10.0 mg |
| | Crystalline cellulose | 25.0 mg |
| | Lactose | 108.5 mg |
| | Magnesium stearate | 1.5 mg |
| | Carboxymethylcellulose calcium | 5.0 mg |
| | Total | 150.0 mg |
| 20 mg formulation | Hydrochloride of general formula (I) compound (wherein R1 represents a hydroxyl group) | 20.0 mg |
| | Crystalline cellulose | 25.0 mg |
| | Lactose | 98.5 mg |
| | Magnesium stearate | 1.5 mg |
| | Carboxymethylcellulose calcium | 5.0 mg |
| | Total | 150.0 mg |

### [Kit Example]

A kit of pharmaceutical composition, which includes (a), a compound represented by the general formula (I): 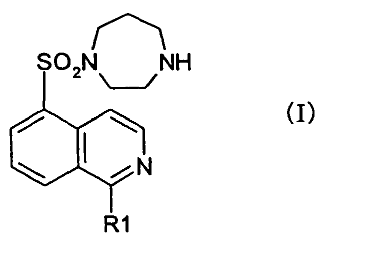 (wherein R1 represents a hydrogen atom or a hydroxyl group) or an acid addition salt or hydrate thereof and which includes (b) can be provided in a form shown below, for example.

At least one of pharmaceutical dosage form units shown in Table 4 and at least one of pharmaceutical dosage form units shown in Table 5 were separately packed in a divided bottle or a divided foil packet, and the bottle or the packet was stored in a package container.

**[Table 4]**

| 1. Ampule agent | | |
|---|---|---|
| | Component | Active ingredient content |
| 10 mg formulation | Hydrochloride of general formula (I) compound (wherein R1 represents a hydrogen atom) | 10 mg |
| 30 mg formulation | Hydrochloride of general formula (I) compound (wherein R1 represents a hydrogen atom) | 30 mg |
| 60 mg formulation | Hydrochloride of general formula (I) compound (wherein R1 represents a hydrogen atom) | 60 mg |
| 10 mg formulation | Hydrochloride of general formula (I) compound (wherein R1 represents a hydroxyl group) | 10 mg |
| 30 mg formulation | Hydrochloride of general formula (I) compound (wherein | 30 mg |
| | R1 represents a hydroxyl group) | |
| 60 mg formulation | Hydrochloride of general formula (I) compound (wherein R1 represents a hydroxyl group) | 60 mg |

| 2. Tablet | | |
|---|---|---|
| | Component | Active ingredient content |
| 10 mg formulation | Hydrochloride of general formula (I) compound (wherein R1 represents a hydrogen atom) | 10.0 mg |
| 20 mg formulation | Hydrochloride of general formula (I) compound (wherein R1 represents a hydrogen atom) | 20.0 mg |
| 10 mg formulation | Hydrochloride of general formula (I) compound (wherein R1 represents a hydroxyl group) | 10.0 mg |
| 20 mg formulation | Hydrochloride of general formula (I) compound (wherein R1 represents a hydroxyl group) | 20.0 mg |

**[Table 5]**

| 1. Ampule agent | | |
|---|---|---|
| | Component | Active ingredient content |
| 10 mg formulation | Nimodipine | 10 mg |

| 2. Tablet | Component | Active ingredient content |
|---|---|---|
| 30 mg formulation | Nimodipine | 30 mg |
| 60 mg formulation | Nimodipine | 60 mg |
| 100 mg formulation | Nimodipine | 100 mg |

| 3. capsule | | |
|---|---|---|
| | Component | Active ingredient content |
| 30 mg formulation | Nimodipine | 30 mg |

### Industrial Applicability

The present invention can provide a pharmaceutical composition that is useful as a preventive or treatment agent for a cerebrovascular disorder.

## Claims

1. A pharmaceutical composition for the prevention or treatment of a cerebrovascular disorder selected from the group consisting of cerebral infarction, cerebral thrombosis, cerebral embolism, cerebral hemorrhage, cerebral vasospasm after subarachnoid hemorrhage, transient ischemic attack, cerebral arteriosclerosis, and cerebral edema, comprising at least one of components (a) and at least one of components (b) shown below:
(a) a compound represented by the general formula (I) 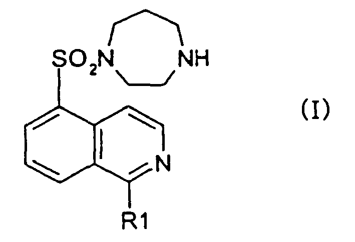 wherein R1 represents a hydrogen atom or an acid addition salt or hydrate thereof; and
(b) nimodipine or a pharmaceutically acceptable salt thereof.

2. A pharmaceutical composition according to claim 1, wherein the cerebrovascular disorder is cerebral vasospasm after subarachnoid hemorrhage.

3. A pharmaceutical composition according to claim 1 or 2, wherein the pharmaceutical composition is a non-mixed combination in form of a kit or a pharmaceutical package.

## Patentansprüche

1. Pharmazeutische Zusammensetzung für die Prophylaxe oder Behandlung einer zerebrovaskulären Störung, die aus der Gruppe ausgewählt wird, welche aus einem Gehirninfarkt, einer cerebralen Thrombose, einer Gehirnembolie, einer Gehirnblutung, einem zerebralen Vasospasmus nach Subarachnoidalblutung, einer transitorischen ischämischen Attacke, einer zerebralen Arteriosklerose und einem Gehirnödem besteht,
umfassend mindestens einen der Bestandteile (a) und mindestens einen der Bestandteile (b), die nachfolgend gezeigt sind:
(a) eine Verbindung, die durch die allgemeine Formel (I) dargestellt wird: 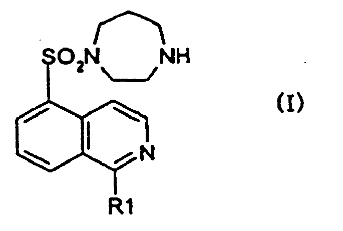 wobei R1 ein Wasserstoffatom darstellt, oder ein Säureadditions-Salz oder Hydrat davon; und
(b) Nimodipin oder ein pharmazeutisch akzeptables Salz desselben.

2. Pharmazeutische Zusammensetzung nach Anspruch 1, wobei die zerebrovaskuläre Störung einen zerebralen Vasospasmus nach Subarachnoidalblutung darstellt.

3. Pharmazeutische Zusammensetzung nach Anspruch 1 oder 2, wobei die pharmazeutische Zusammensetzung eine nicht-gemischte Kombination in Form eines Kits oder einer pharmazeutischen Packung darstellt.

## Revendications

1. Composition pharmaceutique pour la prévention ou le traitement d'un trouble vasculaire cérébral choisi dans le groupe constitué de l'infarctus cérébral, de la thrombose cérébrale, de l'embolie cérébrale, de l'hémorragie cérébrale, du vasospasme cérébral consécutif à une hémorragie sous-arachnoïdienne, de l'accès ischémique transitoire cérébral, de l'athérosclérose cérébrale, et de l'oedème cérébral, comprenant au moins un des composants (a) et au moins un des composants (b) indiqués ci-dessous :
(a) un composé représenté par la formule générale (I) 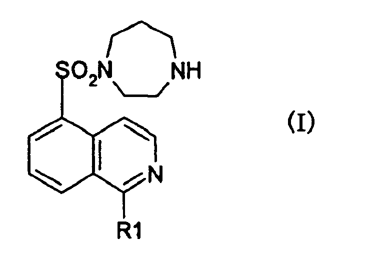 dans laquelle R1 est un atome d'hydrogène ou un sel d'addition d'acide ou hydrate de celui-ci ; et
(b) une nimodipine ou un sel pharmaceutiquement acceptable de celle-ci.

2. Composition pharmaceutique selon la revendication 1, dans laquelle le trouble vasculaire cérébral est le vasospasme cérébral consécutif à une hémorragie sous-arachnoïdienne.

3. Composition pharmaceutique selon la revendication 1 ou 2, dans laquelle la composition pharmaceutique est une combinaison non mélangée sous la forme d'une trousse ou d'un conditionnement pharmaceutique.
